(19) Europäisches Patentamt
European Patent Office
Office européen des brevets

(11) Veröffentlichungsnummer: **0 190 652**
**B1**

(12) # EUROPÄISCHE PATENTSCHRIFT

(45) Veröffentlichungstag der Patentschrift: **16.08.90**

(21) Anmeldenummer: **86101135.1**

(22) Anmeldetag: **29.01.86**

(51) Int. Cl.⁵: **C 12 N 15/15**, C 07 H 21/04, C 12 N 9/99, C 07 K 13/00, C 12 P 21/02, A 61 K 37/02

(54) **Gene für biologisch aktive Proteine.**

(30) Priorität: **08.02.85 DE 3504334**
**13.06.85 DE 3521226**

(43) Veröffentlichungstag der Anmeldung:
**13.08.86 Patentblatt 86/33**

(45) Bekanntmachung des Hinweises auf die Patenterteilung:
**16.08.90 Patentblatt 90/33**

(84) Benannte Vertragsstaaten:
**AT BE CH DE FR GB IT LI LU NL SE**

(56) Entgegenhaltungen:
**EP-A-0 090 505**
**EP-A-0 120 829**

**Thrombosis Research, Vol. 32, 1983 (New York); I. Witt et al.:"Amino Acid Composition and N-terminal Sequence of Antithrombin BM", Seiten 513-518**

(73) Patentinhaber: **HOECHST AKTIENGESELLSCHAFT**
**Postfach 80 03 20**
**D-6230 Frankfurt am Main 80 (DE)**

(72) Erfinder: **Ragg, Hermann, Dr.**
**Am Kirchplatz 14**
**D-6233 Kelkheim (Taunus) (DE)**

(56) Entgegenhaltungen:
**Nature, Vol. 313, 10 Januar 1985 (New York, London); M. Courtney et al.:"Synthesis in E. coli of alpha1-antitrypsin variants of therapeutic potential for emphysema and thrombosis",Seiten 149-151**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 190 652 B1

**Beschreibung**

Die Erfindung betrifft ein neues Mitglied der Genfamilie, die bisher durch die Gene für die Proteinase-Inhibitoren Antithrombin III, $\alpha_1$-Proteinaseinhibitor ($\alpha_1$-Antitrypsin), Antichymotrypsin, Contraspin, $\alpha_1$-Antiplasmin sowie durch Angiotensinogen gekennzeichnet ist. Das gefundene Gen codiert für ein Protein mit der Aminosäuresequenz I (Anhang). Sein codierender Strang weist 2081 Nucleotide auf, wovon sich 636 im Anschluß an das Stop-Codon TAG finden (DNA-Sequenz I). In der Figur ist die "nontranslated region" als weißer Balken, das Strukturgen schraffiert dargestellt. Die DNA-Sequenz I enthält vor dem Codon für die N-terminale Aminosäure (Glycin) noch zwei Nucleotide, die vermutlich für die letzte Aminosäure einer Prosequenz codieren (aus der Analogie zu den Proteinase-Inhibitoren AT BM und Heparin Cofactor II kann geschlossen werden, daß das Glycin die N-terminale Aminosäure des "reifen" neuen Proteinase-Inhibitors ist: I. Witt et al., Thrombosis Research 32 (1983) 513—518; M. J. Griffith et al., J. Biol. Chem. 260 (1950) 2218—2225).

Die Aminosäuresequenz I erlaubt es dem Fachmann, durch Auswahl von Oligopeptiden aus diese Aminosäuresequenz I in bekannter Weise Antikörper herzustellen, damit eine Antikörpersäule zu beladen und mit Hilfe dieser Säule aus biologischem Material das komplette Protein zu isolieren.

Es ist andererseits möglich, mit Hilfe des Gens oder der cDNA-Sequenz I das Protein in geeigneten Wirtsorganismen zu exprimieren.

Es ist weiterhin möglich, aus der Aminosäuresequenz I modifizierte Gene oder Teilgene abzuleiten, die zu modifizierten Proteinen fuhren, in denen einzelne Aminosäuren ausgetauscht, weggelassen oder eingefügt werden. Bei der Konstruktion synthetischer Gene können auch neue Erkennungsstellen für Restriktionsenzyme vorgesehen werden, die weitere Abwandlungen der Aminosäuresequenz erlauben. Bei der Konstruktion synthetischer Gene kann man auch berücksichtigen, daß mikrobielle Wirtsorganismen im Vergleich zu höheren Organismen teilweise andere Codons bevorzugen.

Das neue Protein weist sowohl auf der Ebene der DNA als auch der Aminosäuresequenz Homologien zu den obengenannten Proteinase-Inhibitoren auf. Deshalb eignet sich das Gen auch zur Herstellung von Analogen zu Proteinase-Inhibitoren, indem man die dem "aktiven Zentrum" im Protein entsprechende Nucleotidsequenz des neuen Gens durch die entsprechenden Nucleotidsequenz ersetzt, die für die aktiven Zentren der bekannten Proteinase-Inhibitoren codieren. Das aktive Zentrum des Proteins besteht aus den Aminosäuren Leu-Ser (Aminosäuren 444 und 445 der Aminosäuresequenz I), ihm entspricht die Nucleotidfolge CTG TCC.

Bei einer solchen Konstruktion von Analogen der bekannten Proteinase-Inhibitoren kann man also nach dem "Baukastenprinzip" ein Teilgen I, welches für die Aminosäurefolge vor dem aktiven Zentrum codiert, mit einem Teilgen II, welches für die Aminosäurefolge nach dem aktiven Zentrum codiert, unter Zwischenschaltung eines Genfragments, welches für das entsprechende aktive Zentrum codiert, kombinieren. Bei diesem Vorgehen kann man von den folgenden Erkennungsstellen für Restriktionsenzyme in der DHA-Sequenz I Gebrauch machen:

Sau3A (↓GATC, Positionen 1271—1274), Acc I (GT$^{\downarrow AT}_{\;\;CG}$AC, Positionen 1356—1361) oder Taq I (T↓CGA, Positionen 1246—1249 und 1357—1360).

Die Modifizierung des menschlichen $\alpha_1$-Antitrypsins ist bereits bekannt (S. Rosenberg et al., Nature 312 (1984) 77—80, vgl. A. Maelicke, Nachr.. Chem. Tech. Lab. 32 (1984) 1070; M. Courtney et al., Nature 313 (1985) 149—151), wobei jeweils das aktive Zentrum verändert wurde, die Aminosäuresequenzen vor und nach dem aktiven Zentrum aber unverändert blieben. Erfindungsgemäß werden demgegenübert neue Produkte erhalten, deren Aminosäuresequenzen vor und nach dem aktiven Zentrum dem neuen Proteinase-Inhibitor und gegebenenfalls dessen Modifikationen entsprechen, aber das aktive Zentrum eines anderen Proteinase-Inhibitors enthalten. Die Erfindung erlaubt somit eine vielfältige Abwandlung solcher Proteine die alle die praktisch gleiche Tertiärstruktur besitzen. Ihre biologische Aktivität wird durch das aktive Zentrum beeinflußt.

Das neue Protein und seine Analoga haben proteinaseinhibitorische, insbesondere Thrombininhibitorische, Aktivität und beeinflussen die Blutgerinnung. Sie können deshalb als Arzneimittel in einer Tagesdosis von etwa 0,1 bis 100 mg eingesetzt werden. Da Proteine bekanntermaßen im Magen abgebaut werden, müssen die erfindungsgemäße Proteine in einer Arzneimittelform verabreicht werden, die diesen Abbau vermeidet, bespielsweise durch Verabreichung in Form von magensaftresistenten Kapsel oder durch parenterale Verabreichung.

Die cDNA-Sequenz wurde durch Screening einer menschlichen Leber-cDNA-Bank mit Hilfe des synthetisch gewonnenen Oligonucleotids der DNA-Sequenz II

5′ GGGTTGGCTACTCTGCCCATGAAGA 3′

gewonnen. Diese Nucleotidfolge wurde in Anlehnung an die bekannte cDNA-Sequenz von Proteinase-Inhibitoren nahe dem Carboxyterminus ausgewählt. Es zeigte sich, daß sie weitegehend komplementär zu codierenden Strang entsprechend den Aminosäuren 469 bis 477 der Aminosäuresequenz I ist.

Die Methoden zur Synthese von Oligonucleotiden, zur Hybridiserung und zur Gewennung des Gens sind allgemein bekannt, ebenso das Vorgehen zum Einbringen der genetischen Information in Wirtszellen und die Expression des codierten Polypeptids in prokaryotischen und eukaryotischen Expressionssystemen. Prokaryotische Zellen produzieren das Protein in nicht glykosyliertem Zustand, während eukaryotische Systeme ein glykosyliertes Protein erzeugen können. Eine N-Glykosylierung kann an den Aminosäuren 30, 1690 und 368 der Aminosäuresequenz I stattfinden.

Die Modifikation der DNA-Sequenz I wird im folgenden am Beispiel der Einführung eines anderen aktiven Zentrums erläutert. Hierzu wird die DNA-Sequenz I partiell mit den Endonucleasen Sau3A I and AccI geschnichtten und das kurze DNA-Fragment durch ein synthetisch hergestelltes Genfragment ersetzt, das für die folgenden aktiven Zentren codiert:

| Aminosäure-sequenz | Proteinase-Inhibitor-Aktivität |
|---|---|
| Arg-Ser | wie Antithrombin III (human) |
| Met-Ser | wie $\alpha_1$-Antitrypsin (human) |
| Val-Ser | wie $\alpha_1$-Antitrypsin |
| Lys-Ala | wie Maus-Contraspin |
| Tyr-Ser | wie Maus-$\alpha_1$-Antitrypsin |
| Leu-Met | wie $\alpha_1$-Antiplasmin |

(R. Hill et al., Nature 311 (1984) 175—177; M. Courtney et al., a.a.O.; J. Travis et al., Behring Inst. Mitt. 73 (1983) 56—65).

Das neue Gen eignet sich sommit auch als Ausgangsmaterial zur Herstellung neuer Proteine mit proteinaseinhibitorischer Aktivität. Auch diese neuen Verbindungen können entsprechend den bekannten gleich- bzw. ähnlich wirkenden Proteinaseinhibitoren als Arzneimittel verwendent werden.

Auch diese neuen Analoga bekannter Proteinase-Inhibitoren können weiter abgewandelt werden, indem das Gen in bekannter Weise modifiziert wird, wodurch Proteine erhalten werden, in deren Aminosäurekette Aminosäuren ausgetauscht, weggelassen oder ergänzt sind. Solche Proteine mit Proteinase-Inhibitor-Aktivität sind ebenfalls Gegenstand der Erfindung.

In den folgenden Beispielen werden die einzelnen Aspekte der Erfindung näher erläutert. Prozentangaben beziehen sich auf das Gewicht, sofern keine anderen Angaben gemacht sind.

## Beispiel 1

Isolierung der RNA

Gefrorenes menschliches Leberbiopsiematerial wurde unter flüssigem Stickstoff im Mörser pulverisiert. Das Pulver wurde mit auf 70°C vorgewärmter 4 M Guanidiniumthiocyanat-Stammlösung (J. Chirgwin et al. Biochemistry 18 (1979) 5294—5299) in einem Mixer eine Minute lang homogenisiert. Die Mischung wurde bei 10°C 10 Minuten zentrifugiert (Sorvall-Rotor HB-4 8000 Upm). Die Überstände wurden abgenommen, pro ml Überstand 0,5 g Caesiumchlorid zugesetzt und die Mischung einige Minuten auf 60°C erwärmt. Die erhaltene Lösung wurde anschließend in Ultrazentrifugenröhrchen gefüllt, die zu einem Viertel mit einer Lösung aus 5,7 M Caesiumchlorid, 12,5 mM Natriumsalz der Ethylendiamintetraessigsäure (EDTA, pH 7,5) und 12,5 mM Natriumcitrat (pH 7,0) gefüllt waren. Die Röhrchen wurden bei 20°C 17 Stunden zentrifugiert (Beckman SW-28 Rotor 21000 Upm). Die Überstände wurden abgesaugt und der Niederschlag wurde in TES-Puffer (50 mM Tris-HCl, 10 mM EDTA, 0,2% (w/v) Natriumsalz des Dodecylsulfats (SDS, pH 7.4)) unter Erwärmen suspendiert und einmal mit dem gleichen Volumen einer Mischung aus Chloroform und n-Butanol (4:1, v/v) extrahiert. Nach Zentrifugieren wurde die wäßrige Phase mit einem Zehntel des Volumens 3 M Natriumacetatlösung (pH 5,2) versetzt und mit Ethanol gefällt. Nach mindestens zweistündigen Stehen bei −20°C wurde bei 0°C 20 Minuten zentrifugiert 12 000 Upm und der Niederschlag mit 80%igem Ethanol gewaschen. Das getrocknete Präzipitat wurde in 0,1 molarer Natriumacetatlösung (pH 7,0) gelöst und erneut mit Ethanol gefällt. Das Präzipitat wurde die oben beschrieben zentrifugiert, gewaschen und getrocknet. Nach erneutem Lösen in einer Pufferlösung (0,1 M Natriumacetat, pH 5,2, 10 mM Tris-HCl, pH 7,4, 1 mM EDTA und 0,2% (w/v) SDS) wurde die Lösung zweimal mit dem gleichen Volumen Phenol, äquilibriert mit 1 M Tris-HCl, pH 8,0, und 0,2% (w/v) Hydroxychinolin, extrahiert zentrifugiert. Nach einmaliger Extraktion mit dem gleichen Volumen Chloroform und Zentrifugieren wurde die wäßrige Phase mit dem gleichen Volumen einer Lösung aus Harnstoff (8 M) und Lithiumchlorid (4 M) versetzt und über nacht bei 0°C aufbewahrt. Nach 20 Minuten Zentrifugieren bei 0°C (Rotor SS 34, 10 000 Upm) wurde der Niederschlag in TES-Puffer aufgenommen, mit einem Zehntel des Volumens an 3 M Natriumacetatlösung (pH 5,6) versetzt und mit Ethanol gefällt. Der Niederschlag wurde wie beschrieben zentrifugiert, gewaschen und getrocknet und schließlich in Pufferlösung aufgenommen (50 nM Tris-HCl, 1 mM EDTA, 0,1% (w/v) SDS pH 7,5). Die Mischung wurde mit dem gleichen Volumen an 1 M Kochsalzlösung versetzt und die Poly(A)-RNA durch zweimalige Chromatographie an Oligo-(dT)-Cellulose isoliert. Die Poly(A)-RNA wurde nach Zugabe von Natriumacetatlösung mit Ethanol gefällt und schließlich in einer Konzentration von 1 µg/µl Pufferlösung (1 mM Tris-HCl, 0,1 mM EDTA, pH 7,0) gelöst.

Beispiel 2

Synthese, Klonierung und Screening der cDNA

10 µg Poly(A)-RNA in 10 µl des vorstehend benannten Puffers wurden 3 Minuten auf 70°C erwärmt, in Eiswasser abgeschreckt und zur Synthese der cDNA eingesetzt. Die Synthese des ersten Stranges in 100 µl Endvolumen erfolgte nach der Methode von M. Wickens et al., J. Biol. Chem. 253 (1978) 2483—2495 unter Verwendung von $\alpha^{32}$P-markiertem Thymidintriphosphat, wobei zusätzlich der RNase-Inhibitor RNasin (100 U pro Ansatz; Firma Biotec) engesetzt wurde. Bei der Synthese des zweiten Stranges (ebenfalls nach Wickens et al., a.a.O.) wurde das Klenow-Fragment der DNA-Polymerase von E. coli (100 U/200 µl-Reaktion) verwendet und die Reaktion 4 Stunden bei 15°C und anschließend 2 Stunden bei 20°C durchgeführt. Durch Extraktion mit einer Mischung aus gleichen Volumina Phenol und Chloroform wurde die Reaktion gestoppt. Die wäßrige Phase wurde nach Zentrifugieren über eine 5 ml-Säule mit ®SEPHADEX H-100, äquilibriert in 20 mM Kochsalz, fraktioniert. Die cDNA enthaltenden Fraktionen wurden gesammelt, mit Natriumacetat versetzt und über Nacht mit Ethanol gefällt. Zur Entfernung der "Hairpin"-Strukturen wurde die dsDNA 30 Minuten bei 37°C mit 90 U Nuclease $S_1$ (P-L Biochemicals) in 250 µl Reaktionsvolumen behandelt (Maniatis et al., Molecular Cloning, a Laboratory Manual; Cold Spring Harbor, 1982). Die Reaktion wurde durch Extraktion mit einer Mischung aus gleichen Volumina Phenol und Chloroform gestoppt und die DNA mach Zugabe von Natriumacetat mit Ethanol gefällt. Die Enden der dsDNA wurden in bekannter Weise mit Hilfe von Klenow-Polymerase (Maniatis et al., a.a.O.) repariert, die Reaktion durch Extraktion mit der Mischung aus gleichen Volumina Phenol und Chloroform gestoppt und die DNA nach Zugabe von Natriumacetat mit Ethanol gefällt.

Zur Transformation von E. coli HB 101 wurde das Plasmid pUC13 (BRL, Catalogue & Reference Guide, 1983, S. 89) nach Standardmethoden mit der Restriktionsendonuclease Sma I gespalten und mit alkalischer Phosphatase behandelt. Die gewonnene ds-cDNA wurde ohne weitere Vorbehandlung in Puffer (1 mM Tris-HCl, pH 7,5, 0,1 mM EDTA) gelöst und in den mit Sma I gespaltenen und phosphatierten Vektor pUC13 ligiert.

Die Transformation in E. coli HB 101 erfolgte nach beschriebenen Methoden (Maniatis et al., a.a.O.). Ampicillinresistente Kolonien wurden auf Nitrocellulosefiltern angezogen. Replica-Platten wurden mig 150 µg/ml Chloramphenicol behandelt und die Kolonien lysiert. Die Fixierung der DNA auf die Nitrocellulosefilter wurde in bekannter Weise (Maniatis et al., a.a.O.) durchgeführt.

Zur in situ-Hybridisierung wurden 52 Nitrocellulosefilter mit jeweils 300 bis 400 Kolonien 2 Stunden bei 42°C in "prewashing solution" (Maniatis et al., a.a.o., S. 326) gewaschen und anschließend je 26 Filter je 6 Stunden bei 42°C in 150 ml der folgenden Mischung vorhybridisiert:

0,9 M NaCl
0,18 M Tris-HCl, pH 8,0
6 mM EDTA
5-fach konzentrierte Denhardt-Lösung
0,2% (w/v) SDS
200 µg/ml gescherchte und denaturierte Kalbsthymus-DNA (Sigma)
200 µ/ml Hefe/RNA (Sigma)
0,5% (v/v) nichtionisches Tensid (Nonidet P-40, Sigma)

Die Hybridisierung mit $10^5$ cpm/ml der Probe mit der DNA-Sequenz II erfolgte mit dem gleichen Puffer (16 Stunden bei 42°C). Die Filter wurden anschließend 2 × 20 Minuten bei Raumtemperatur gewaschen (0,9 M Kochsalz, 0,09 M Natriumcitrat, pH 7,0) und anschließend 2 × 20 Minuten bei 33°C in dem gleichen Puffer gewaschen. Die Filter wurden hierauf getrocknet und damit Röntgenfilme bei −70°C exoniert.

Die Probe mit der DNA-Sequenz II wurde nach dem Phosphotriesterverfahren synthetisiert. Zur Hybridisierung wurde das Oligonucleotid am 5'-Ende mit $^{32}$P markiert.

Bei dem Screening wurde ein rekombinantes Plasmid (pL 10/2) isoliert, das sehr stark mit dem Oligonucleotid der DNA-Sequenz II hybridisierte und ca. 1500 bp menschliche DNA umfaßte. Die Nucleotidsequenz des cDNA-Inserts wurde nach der Methode von Maxam und Gilbert (Methods in Enzymology 65 (1980) 499—560) also Nucleotide 518—2081 der DNA-Sequenz I bestimmt.

Zur 5'-Verlängerung des Klons pL 10/2 erfolgte eine erneute cDNS-Synthese mit der Nach Beispiel 1 erhaltenen Poly(A)-RNA:

Nach dem Phosphortriesterverfahren synthetisiert man das Oligonucleotid der DNA-Sequenz III

3' AGCTTCGCGTTGACTGTGGGGCCC (III)

die der Nucleotidfolge 1051—1064 des nicht-codierenden Stranges der DNA-Sequenz I entspricht.

500 pmol des mit Polynucleotid-Kinase und $\gamma$-$^{32}$P-markiertem ATP umgesetzten Oligonucleotids III und 10 µg Poly(A)-RNA nach Beispiel 1 wurden in 52 µl Puffer (1 mM Tris-HCl, pH 7,5; 0,1 mM EDTA; 0,5 M KCl) 3 Minuten lang auf 70°C erwärmt und anschließend innerhalb von 30 Minuten auf 43°C abgekühlt. Nach der

Methode von Gubler und Hoffman, Gene 25 (1983) 263—269, wurde die cDNA synthetisiert, wobei das Oligonucleotid III also Primer dient. Die Enden der doppelsträngigen cDNA wurden mit Hilfe von T4-DNA-Polymerase (PL-Biochemicals) nach der Methode von Toole et al., Nature 312 (1984) 342—347, repariert.

Nach Anfügen von mit Polynucleotid-Kinase und γ-$^{32}$P-markiertem ATP umgesetzten Hind III-Linkern der Formel IV

5′ pGCAAGCTTGC 3′ (IV)

(BRL) und nach Spalten mit Hind III wurde die cDNA in den Vektor pAT 153 (A. J. Twigg und D. Sherratt, Nature 283 (1980) 216—218) liegiert, der zuvor mit Hind III und alkalischer Phosphatase aus Kälberdarm (Boehringer Mannheim) behandelt wurde. Die Transformation von E. coli HB 101, die Amplifikation mit Chloramphenicol und die Lyse der Kolonien erfolgte wie vorstehend beschrieben.

Die in situ-Koloniehybridisierung erfolgte wie vorstehend ausgeführt, wobei als Hybridisierungsprobe ein 788 bp DNA-Fragment verwendet wurde, das mit Hilfe des Restriktionsenzyms Acc I aus dem Plasmid pL 10/2 ausgeschnitten wurde und durch Nicktranslation (Maniatis et al., a.a.O.) zu einer spezifischen Radioaktivität von mindestens $10^8$ cpm/μg markiert wurde. Die Filter wurden jeweils 15 Minuten lang wie folgt gewaschen:

| | | | |
|---|---|---|---|
| bei | Raumtemperatur | in | 6 × SSC |
| bei | 42°C | in | 2 × SSC |
| bei | 50°C | in | 1 × SSC |
| bei | 50°C | in | 0,1 × SSC |

1 × SSC = 0,15 M NaCl, 15 mM Natriumcitrat, pH 7,0

Durch das Screening wurden 2 stark hybridisierende Klone identifiziert. Einer der Klone (pH 14) enthielt ein Insert, das ca. 1060 bp Human-DNA umfaßte. Die Nucleotidsequenz dieses cDNA-Inserts wurde nach dem Verfahren von Maxam und Gilbert, a.a.O., bestimmt.

Durch Vergleich der cDNA-Sequenzen der Klone pH 14 und pL 10/2 wurde festgestellt, daß pH 14 die 5′-terminale Verlängerung von pL 10/2 — im Anschluß an eine Überlappung von etwa 480 bp — enthält (Figur). pH 14 enthält die Nucleotide 1—1062 der DNA-Sequenz I.

## Beispiel 3

Expression der cDNA

Das Plasmid pH 14 wird mit Hind III und partiell mit BamHI geschnitten und das 841 bp Hind III-BamHI-Fragment nach Elektrophorese auf einem 1,4%igen Agarosegel (Low Gel Temperature Agarose, BIO-RAD) isoliert.

Das Plasmid pL 10/2 wird mit Hind III und BamHI geschnitten und das 4 kb Hind III-BamHI-Fragment mit dem 840 bp-Fragment aus Plasmid pH 154 ligiert. Diese Konstruktion (Plasmid phLS2) kodiert für einen Proteinaseinhibitor, der am N-Terminus um 9 Aminosäuren verlängert ist. Dieses Expressionsplasmid erlaubt nach Transformation in E. coli und Induktion mit Isopropyl-β-D-thiogalactopyranosid die Synthese des verlängerten Proteinaseinhibitors unter der Kontrolle des lac-Promotor-Operator-Systems.

Aminosäure-Sequenz I:

```
1                                            10                                                20
Gly Ser Lys Gly Pro Leu Asp Gln Leu Glu Lys Gly Gly Glu Thr Ala Gln Ser Ala Asp Pro
                                    30                                             40
Gln Trp Glu Gln Leu Asn Asn Lys Asn Leu Ser Met Pro Leu Leu Pro Ala Asp Phe His Lys
                                50                                             60
Glu Asn Thr Val Thr Asn Asp Trp Ile Pro Glu Gly Glu Glu Asp Asp Asp Tyr Leu Asp Leu
                            70                                             80
Glu Lys Ile Phe Ser Glu Asp Asp Asp Tyr Ile Asp Ile Val Asp Ser Leu Ser Val Ser Pro
                        90                                             100
Thr Asp Ser Asp Val Ser Ala Gly Asn Ile Leu Gln Leu Phe His Gly Lys Ser Arg Ile Gln
                    110                                            120
Arg Leu Asn Ile Leu Asn Ala Lys Phe Ala Phe Asn Leu Tyr Arg Val Leu Lys Asp Gln Val
                130                                            140
Asn Thr Phe Asp Asn Ile Phe Ile Ala Pro Val Gly Ile Ser Thr Ala Met Gly Met Ile Ser
            150                                            160
Leu Gly Leu Lys Gly Glu Thr His Glu Gln Val His Ser Ile Leu His Phe Lys Asp Phe Val
    170                                            180
Asn Ala Ser Ser Lys Tyr Glu Ile Thr Thr Ile His Asn Leu Phe Arg Lys Leu Thr His Arg
190                                            200                                            210
Leu Phe Arg Arg Asn Phe Gly Tyr Thr Leu Arg Ser Val Asn Asp Leu Tyr Ile Gln Lys Gln
```

```
                                                220                                         230
Phe Pro Ile Leu Leu Asp Phe Lys Thr Lys Val Arg Glu Tyr Tyr Phe Ala Glu Ala Gln Ile
                                240                                         250
Ala Asp Phe Ser Asp Pro Ala Phe Ile Ser Lys Thr Asn Asn His Ile Met Lys Leu Thr Lys
                            260                                         270
Gly Leu Ile Lys Asp Ala Leu Glu Asn Ile Asp Pro Ala Thr Gln Met Met Ile Leu Asn Cys
                        280                                         290
Ile Tyr Phe Lys Gly Ser Trp Val Asn Lys Phe Pro Val Glu Met Thr His Asn His Asn Phe
                    300                                         310
Arg Leu Asn Glu Arg Glu Val Val Lys Val Ser Met Met Gln Thr Lys Gly Asn Phe Leu Ala
                320                                         330
Ala Asn Asp Gln Glu Leu Asp Cys Asp Ile Leu Gln Leu Glu Tyr Val Gly Gly Ile Ser Met
            340                                         350
Leu Ile Val Val Pro His Lys Met Ser Gly Met Lys Thr Leu Glu Ala Gln Leu Thr Pro Arg
        360                                         370
Val Val Glu Arg Trp Gln Lys Ser Met Thr Asn Arg Thr Arg Glu Val Leu Leu Pro Lys Phe
    380                                         390
Lys Leu Glu Lys Asn Tyr Asn Leu Val Glu Ser Leu Lys Leu Met Gly Ile Arg Met Leu Phe
400                                         410                                         420
Asp Lys Asn Gly Asn Met Ala Gly Ile Ser Asp Gln Arg Ile Ala Ile Asp Leu Phe Lys His
```

```
                                    430                                               440
Gln Gly Thr Ile Thr Val Asn Glu Glu Gly Thr Gln Ala Thr Thr Val Thr Thr Val Gly Phe
                                450                                               460
Met Pro Leu Ser Thr Gln Val Arg Phe Thr Val Asp Arg Pro Phe Leu Phe Leu Ile Tyr Glu
                            470                                               480
His Arg Thr Ser Cys Leu Leu Phe Met Gly Arg Val Ala Asn Pro Ser Arg Ser
```

DNA-Sequenz I

```
1                                                                        50
 GT GGG AGC AAA GGC CCG CTG GAT CAG CTA GAG AAA GGA GGG GAA ACT GCT CAG TCT GCA GAT
                                                100
CCC CAG TGG GAG CAG TTA AAT AAC AAA AAC CTG AGC ATG CCT CTT CTC CCT GCC GAC TTC CAC
                                    150
AAG GAA AAC ACC GTC ACC AAC GAC TGG ATT CCA GAG GGG GAG GAG GAC GAC GAC TAT CTG GAC
            200                                                                 250
CTG GAG AAG ATA TTC AGT GAA GAC GAC GAC TAC ATC GAC ATC GTC GAC AGT CTG TCA GTT TCC
                                                                    300
CCG ACA GAC TCT GAT GTG AGT GCT GGG AAC ATC CTC CAG CTT TTT CAT GGC AAG AGC CGG ATC
                                                350
CAG CGT CTT AAC ATC CTC AAC GCC AAG TTC GCT TTC AAC CTC TAC CGA GTG CTG AAA GAC CAG
                                400
GTC AAC ACT TTC GAT AAC ATC TTC ATA GCA CCC GTT GGC ATT TCT ACT GCG ATG GGT ATG ATT
            450                                                             500
TCC TTA GGT CTG AAG GGA GAG ACC CAT GAA CAA GTG CAC TCG ATT TTG CAT TTT AAA GAC TTT
                                                            550
GTT AAT GCC AGC AGC AAG TAT GAA ATC ACG ACC ATT CAT AAT CTC TTC CGT AAG CTG ACT CAT
                                                600
CGC CTC TTC AGG AGG AAT TTT GGG TAC ACA CTG CGG TCA GTC AAT GAC CTT TAT ATC CAG AAG
```

```
                  650
CAG TTT CCA ATC CTG CTT GAC TTC AAA ACT AAA GTA AGA GAG TAT TAC TTT GCT GAG GCC CAG
    700                                                              750
ATA GCT GAC TTC TCA GAC CCT GCC TTC ATA TCA AAA ACC AAC AAC CAC ATC ATG AAG CTC ACC
                                                    800
AAG GGC CTC ATA AAA GAT GCT CTG GAG AAT ATA GAC CCT GCT ACC CAG ATG ATG ATT CTC AAC
                                        850
TGC ATC TAC TTC AAA GGA TCC TGG GTG AAT AAA TTC CCA GTG GAA ATG ACA CAC AAC CAC AAC
                        900
TTC CGG CTG AAT GAG AGA GAG GTA GTT AAG GTT TCC ATG ATG CAG ACC AAG GGG AAC TTC CTC
    950                                                          1000
GCA GCA AAT GAC CAG GAG CTG GAC TGC GAC ATC CTC CAG CTG GAA TAC GTG GGG GGC ATC AGC
                                                    1050
ATG CTA ATT GTG GTC CCA CAC AAG ATG TCT GGG ATG AAG ACC CTC GAA GCG CAA CTG ACA CCC
                                    1100
CGG GTG GTG GAG AGA TGG CAA AAA AGC ATG ACA AAC AGA ACT CGA GAA GTG CTT CTG CCG AAA
                    1150
TTC AAG CTG GAG AAG AAC TAC AAT CTA GTG GAG TCC CTG AAG TTG ATG GGG ATC AGG ATG CTG
    1200                                                         1250
TTT GAC AAA AAT GGC AAC ATG GCA GGC ATC TCA GAC CAA AGG ATC GCC ATC GAC CTG TTC AAG
                                                1300
CAC CAA GGC ACG ATC ACA GTG AAC GAG GAA GGC ACC CAA GCC ACC ACT GTG ACC ACG GTG GGG
```

```
                                     1350
TTC ATG CCG CTG TCC ACC CAA GTC CGC TTC ACT GTC GAC CGC CCC TTT CTT TTC CTC ATC TAC
                    1400
GAG CAC CGC ACC AGC TGC CTG CTC TTC ATG GGA AGA GTG GCC AAC CCC AGC AGG TCC TAG

AGGTGGAGGTCTAGGTGTCTGAAGTGCCTTGGGGGCACCCTCATTTTGTTTCCATTCCAACAACGAGAACAGAGA

TGTTCTGGCATCATTTACGTAGTTTACGCTACCAATCTGAATTCGAGGCCCATATGAGAGGAGCTTAGAAACGACCAAG

AAGAGAGGCTTGTTGGAATCAATTCTGCACAATAGCCCATGCTGTAAGCTCATAGAAGTCACTGTAACTGTAGTGTGTC

TGCTGTTACCTAGAGGGTCTCACCTCCCCACTCTTCACAGCAAACCTGAGCAGCGCGTCCTAAGCACCTCCCGCTCCGG

TGACCCCATCCTTGCACACCTGACTCTGTCACTCAAGCCTTTCTCCACCAGGCCCCTCATCTGAATACCAAGCACAGAAA

TGAGTGGTGTGACTAATTCCTTACCTCTCCCAAGGAGGGTACACAACTAGCACCATTCTTGATGTCCAGGGAAGAAGCCA

CCTCAAGACATATGAGGGGTGCCCTGGGCTAATGTTAGGGCTTAATTTTCTCAAAGCCTGACCTTTCAAATCCATGATG

AATGCCATCAGTCCCTCCTGCTGTTGCCTCCCTGTGACCTGGAGGACAGTGTGTGCCATGTCTCCCATACTAGAGATAA

ATAAAT
```

**Patentansprüche für die Vertragsstaaten: BE CH DE FR GB IT LI LU NL SE**

1. cDNA, codierend für das Protein der Aminosäuresequenz I gemäß Beschreibung.
2. cDNA, erhältlich durch Screening einer cDNA-Genbank aus humanem Leber-Biopsiematerial unter Verwendung einer Probe der DNA-Sequenz II

5' GGGTTGGCTACTCTGCCCATGAAGA 3'

und codierend für ein Analogon des Proteins der Aminosäuresequenz I mit Proteinase-Inhibitor-Aktivität.
3. cDNA, gekennzeichnet durch die DNA-Sequenz I gemäß Beschreibung.
4. cDNA, gekennzeichnet durch die Nucleotide 1 bis etwa 1330 und von etwa 130 vis 1442 der DNA-Sequenz I sowie einer DNA-Sequenz mit den Codons für Arg-Ser, Met-Ser, Val-Ser, Lys-Ala, Tyr-Ser oder Leu-Met, die im Leserahmen zwischen den beiden Nucleotidsequenzen angeordnet ist.
5. cDNA nach einem oder mehreren der vorhergehenden Ansprüche, zusätzlich codierend für ein Signalpeptid.
6. Protein mit Proteinase-Inhibitor-Aktivität, gekennzeichnet durch die Aminosäuren 1 bis 443 und 446 bis 480 der Aminosäure-Sequenz I, insbesondere enthaltend die Aminosäuresequenz I.
7. DNA-Sequenzen, codierend für Proteine nach Anspruch 6.
8. Verfahren zur Herstellung von ggf. glykosylierten Proteinen, dadurch gekennzeichnet, daß man eine DNA gemäß Anspruch 1 bis 5 oder 7 in ein Expressionssystem einbringt und dort zur Expression bringt.
9. Verfahren zur Isolierung des nach Anspruch 8 erhaltenen Proteins oder Glycoproteins, dadurch gekennzeichnet, daß man das Rohprotein durch Affinitätschromatographie mit Hilfe von Antikörpern gewinnt, die durch Immunisieren mit Oligopeptiden entsprechend der Aminosäuresequenz I gewonnen wurden.
10. Arzneimittel, gekennzeichnet durch einen Gehalt an einem Protein nach Anspruch bzw. einem nach Anspruch 8 oder 9 erhaltenen Protein.

**Patentansprüche für den Vertragsstaat: AT**

1. Verfahren zur Herstellung von ggf. glykosylierten Proteinen, dadurch gekennzeichnet, daß man eine cDNA, codierend für das Protein der Aminosäuresequenz I gemäß Beschriebung in ein Expressionssystem einbringt und dort zur Expression bringt.
2. Verfahren nach Anspruch 1, dadurch gekennzeichnet, daß man eine cDNA einsetzt, die durch Screening einter cDNA-Genbank aus humanem Leber-Biopsiematerial unter Verwendung einer Probe der DNA-Sequenz II

5' GGGTTGGCTACTCTGCCCATGAAGA 3'

erhältlich ist und für ein Analogon des Proteins der Aminosäuresequenz I mit Proteinase-Inhibitor-Aktivität codiert.
3. Verfahren nach Anspruch 1 oder 2, dadurch gekennzeichnet, daß die cDNA die DNA-Sequenz I gemäß Beschreibung hat.
4. Verfahren nach einem oder mehreren der vorhergehenden Ansprüche, dadurch gekennzeichnet, daß die cDNA die Nucleotide 1 bis etwa 1330 und von etwa 1340 bis 1442 der DNA-Sequenz I sowie eine DNA-Sequenz mit den Codons für Arg-Ser, Met-Ser, Val-Ser, Lys-Ala, Tyr-Ser oder Leu-Met enthält, die im Leserahmen zwischen den beiden Nucleotidsequenzen angeordnet ist.
5. Verfahren nach einem oder mehreren der vorhergehenden Ansprüche, dadurch gekennzeichnet, daß die cDNA zusätzlich für ein Signalpeptid codiert.
6. Verfahren nach einem oder mehreren der vorhergehenden Ansprüche, dadurch gekennzeichnet, daß man ein Protein mit Proteinase-Inhibitor-Aktivität mit den Aminosäuren 1 bis 443 und 446 bis 480 der Aminosäure-Sequenz I herstellt.
7. Verfahren nach einem oder mehreren der vorhergehenden Ansprüche, dadurch gekennzeichnet, daß man ein Protein, enthaltend die Aminosäuresequenz I, herstellt.
8. Verfahren zur Isolierung des nach einem oder mehreren der Ansprüche 1 bis 7 erhaltenen Proteins oder Glycoproteins, dadurch gekennzeichnet, daß man das Rohprotein durch Affinitätschromatographie mit Hilfe von Antikörpern gewinnt, die durch Immunisieren mit Oligopeptiden entsprechend der Aminosäuresequenz I gewonnen wurden.
9. Verfahren zur Herstellung eines Arzneimittels, dadurch gekennzeichnet, daß man ein nach einem oder mehreren der vorhergehenden Ansprüche erhaltenese Protein in eine zur Darreichung geeignete Form bringt.

**Revendications pour les Etats contractants: BE CH DE FR GB IT LI LU NL SE**

1. ADNc codant pout la protéine ayant la séquence d'aminoacides I selon la description.

2. ADNc pouvant être obtenu par criblage d'une banque de gènes d'ADNc provenant de matière de biopsie de foie humain, avec utilisation d'une sonde ayant la séquence d'ADN II

5' GGGTTGGCTACTCTGCCCATGAAGA 3'

et codant pour un analogue de la protéine ayant la séquence d'aminoacides I et à activité inhibitrice de protéinase.

3. ADNc caractérisé par la séquence d'ADN I selon la description.

4. ADNc caractérisé par les nucléotides 1 à environ 1330 et d'environ 130 à 1442 de la séquence d'ADN I, ainsi que par une séquence d'ADN comportant les codons pour Arg-Ser, Met-Ser, Val-Ser, Lys-Ala, Tyr-Ser ou Leu-Met, qui, dans le cadre de lecture, est disposée entre les deux séquences nucléotidiques.

5. ADNc seon une ou plusieurs des revendications précédentes, codant en outre pour un peptide signal.

6. Protéine à activité inhibitrice de protéinase, caractérisé par les aminoacides 1 à 443 et 446 à 480 de la séquence d'aminoacides I, en particulier contenant la séquence d'aminoacides I.

7. Séquence d'ADN codant pour des protéines selon la revendication 6.

8. Procédé pour la production de protéines éventuellement glycosylées, caractérisé en ce que l'on introduit dans un système d'expression un ADN selon l'une des revendications 1 à 5 ou 7 et on l'exprime dans celui-ci.

9. Procédé pour l'isolement de la protéine ou glycoprotéine obtenue selon la revendication 8, caractérise en ce que l'on obtient la protéine brute par chromatographie d'affinité à l'aide d'anticorps qui ont été obtenus par immunisation avec des oligopeptides correspondant à la séquence d'aminoacides I.

10. Médicament, caractérisé par une teneur en une protéine selon la revendication 6 ou en une protéine obtenue selon la revendication 8 ou 9.

**Revendications pour l'Etat contractant: AT**

1. Procédé pour la production de protéines éventuellement glycosylées, caractérisé en ce que l'on introduit dans le système d'expression un ADNc codant pour la protéine ayant la séquence d'aminoacides I selon la description et on l'exprime dans celui-ci.

2. Procédé selon la revendication 1, caractérisé en ce que l'on utilise un ADNc qui peut être obtenu par criblage d'une banque de gènes d'ADNc provenant de matière de biopsie de foie humain, avec utilisation d'une sonde ayant la séquence d'ADN II

5' GGGTTGGCTACTCTGCCCATGAAGA 3'

et codant pour un analogue de la protéine ayant la séquence d'aminoacides I et à activité inhibitrice de protéinase.

3. Procédé selon la revendication 1 ou 2, caractérisé en ce que l'ADNc a la séquence d'ADN I selon la description.

4. Procédé selon une ou plusieurs des revendications précédentes, caractérisé en ce que l'ADNc contient les nucléotides 1 à environ 1330 et d'environ 1340 à 1442 de la séquence d'ADN I, ainsi qu'une séquence d'ADN, comportant les codons pour Arg-Ser, Met-Ser, Val-Ser, Lys-Ala, Tyr-Ser ou Leu-Met, qui, dans le cadre de lecture, est disposée entre les deux séquences nucléotidiques.

5. Procédé selon une ou plusieurs des revendications précédentes, caractérisé en ce que l'ADN code en outre pour un peptide signal.

6. Procédé selon une ou plusieurs des revendications précédentes, caractérisé en ce que l'on produit une protéine à activité inhibitrice de protéinase, avec les aminoacides 1 à 443 et 446 à 480 de la séquence d'aminoacides I.

7. Procédé selon une ou plusieurs des revendications précédentes, caractérisé en ce que l'on produit une protéine contenant la séquence d'aminoacides I.

8. Procédé pour l'isolement de la protéine ou glycoprotéine obtenue selon une ou plusieurs des revendications 1 à 7, caractérisé en ce que l'on obtient la protéine brute par chromatographie d'affinité à l'aide d'anticorps qui ont été obtenus par immunisation avec des oligopeptides correspondant à la séquence d'aminoacides I.

9. Procédé pour la fabrication d'un médicament, caractérisé en ce que l'on met sous une forme appropriée à l'administration une protéine obtenue selon une ou plusieurs des revendications précédentes.

**Claims for the Contracting States: BE CH DE FR GB IT LI LU NL SE**

1. cDNA coding for the protein of amino acid sequence I according to the description.

2. cDNA obtainable by screening of a cDNA gene bank of human liver biopsy material using a probe of DNA sequence II

5' GGGTTGGCTACTCTGCCCATGAAGA 3'

and coding for an analog of the protein of amino acid sequence I with proteinase inhibitor activity.

3. cDNA characterized by DNA sequence I as defined in the description.

4. cDNA characterized by nucleotides 1 to about 1330 and from about 1340 to 1442 of DNA sequence I, as well as a DNA sequence which has the codons of Arg-Ser, Met-Ser, Val-Ser, Lys-Ala, Tyr-Ser or Leu-Met and which is located in the reading frame between the two nucleotide sequences.

5. cDNA according to one or more of the preceding claims, additionally coding for a signal peptide.

6. Protein with proteinase inhibitor activity, characterized by amino acids 1 to 443 and 446 to 480 of amino acid sequence I, in particular containing amino acid sequence I.

7. DNA sequences coding for proteins according to claim 6.

8. A process for the preparation of proteins which are glycosylated where appropriate, characterized in that a DNA according to any one of claims 1 to 5 or 7 is introduced into an expression system and expressed therein.

9. A process for the isolation of the protein or glycoprotein obtained according to claim 8, characterized by obtaining the crude protein by affinity chromatography with the aid of antibodies which have been obtained by immunization with oligopeptides corresponding to amino acid sequence I.

10. A medicament characterized by a protein according to claim 6 or a protein obtained according to claim 8 or 9.

**Claims for the Contracting State: AT**

1. A process for the preparation of proteins, which are glycosylated where appropriate, characterized in that a cDNA coding for the protein of amino acid sequence I according to the description is introduced into an expression system and expressed therein.

2. The process according to claim 1, wherein use is made of a cDNA which is obtainable by screening of a CDNA gene bank of human liver biopsy material using a probe of DNA sequence II

5′ GGGTTGGCTACTCTGCCCATGAAGA 3′

and codes for an analog of the protein of amino acid sequence I with proteinase inhibitor activity.

3. The process according to claim 1 or 2, characterized in that the cDNA has the DNA sequence I according to the description.

4. The process according to one or more of the preceding claims, characterized in that the cDNA contains the nucleotides 1 to about 1330 and from about 1340 to 1442 of DNA sequence I, as well as a DNA sequence which contains the codons for Arg-Ser, Met-Ser, Val-Ser, Lys-Ala, Tyr-Ser or Leu-Met and which is located in the reading frame between the two nucleotide sequences.

5. The process according to one or more of the preceding claims, characterized in that the cDNA additionally codes for a signal peptide.

6. The process according to one or more of the preceding claims, characterized in that a protein having proteinase inhibitor activity and having amino acids 1 to 443 and 446 to 480 of amino acid sequence I is prepared.

7. The process according to one or more of the preceding claims, characterized in that a protein containing amino acid sequence I is prepared.

8. A process for the isolation of the protein or glycoprotein obtained according to one or more of claims 1 to 7, characterized by obtaining the crude protein by affinity chromatography with the aid of antibodies which have been obtained by immunization with oligopeptides corresponding to amino acid sequence I.

9. A process for the production of a medicament, characterized by converting a protein obtained according to one or more of the preceding claims into a suitable form for administration.

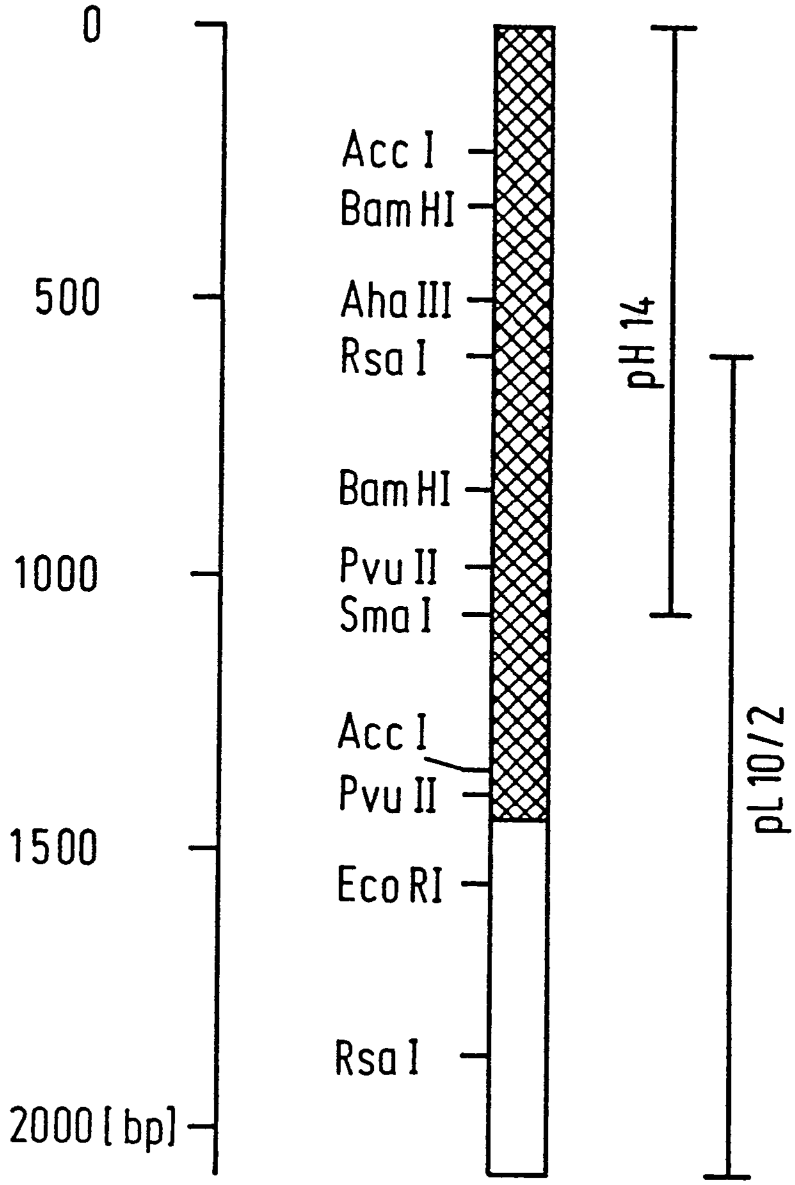
0
500
1000
1500
2000 [bp]
Acc I
Bam HI
Aha III
Rsa I
Bam HI
Pvu II
Sma I
Acc I
Pvu II
Eco RI
Rsa I
pH 14
pL 10/2